# EUROPÄISCHE PATENTANMELDUNG

(11) **EP 0 545 171 A1**
(43) Veröffentlichungstag der Anmeldung: **09.06.1993**
(21) Anmeldenummer: 92119777.8
(22) Anmeldetag: 20.11.1992
(51) Int. Cl.: C07D 215/14, A61K 31/47

(54) **Chinolymethoxyphenylessigsäure-acylamide und -harnstoffe**

(30) Priorität: 03.12.1991 DE 4139750
(71) Anmelder: BAYER AG, 51368 Leverkusen (DE)
(72) Erfinder: Raddatz, Siegfried, Dr., W-5000 Köln 80 (DE); Mohrs, Klaus-Helmut, Dr., W-5600 Wuppertal 1 (DE); Matzke, Michael, Dr., W-5600 Wuppertal 1 (DE); Fruchtmann, Romanis, W-5000 Köln 60 (DE); Hatzelmann, Armin, Dr., W-7750 Konstanz (DE); Müller-Peddinghaus, Reiner, Prof. Dr., W-5060 Bergisch Gladbach (DE)

(57) **Zusammenfassung**

Chinolylmethoxyphenylessigsäure-acylamide und -harnstoffe können hergestellt werden, indem man entweder entsprechende Carbonsäuren mit Amiden oder entsprechende Amide mit Isocyanaten oder Harnstoffen umsetzt. Die Chinolylmethoxyphenylessigsäure-acylamide oder -harnstoffe können als Wirkstoffe in Arzneimitteln verwendet werden.

## Beschreibung

Die Erfindung betrifft Chinolylmethoxyphenylessigsäure-acylamide und -harnstoffe, Verfahren zu ihrer Herstellung und ihre Verwendung in Arzneimitteln.

Substituierte 4-(Chinolin-2-yl-methoxy)phenylessigsäurederivate und α-substituierte 4-(Clinolin-2-yl-methoxy)phenylessigsäurederivate sind aus EP 344 519 (US 4 970 215) und EP 339 416 bekannt.

Außerdem sind substituierte (Chinolin-2-yl-methoxy)phenylcarbonylharnstoffe aus der EP 428 860 bekannt.

Die vorliegende Erfindung betrifft Chinolylmethoxyphenylessigsäure-acylamide und -harnstoffe der allgemeinen Formel (I) 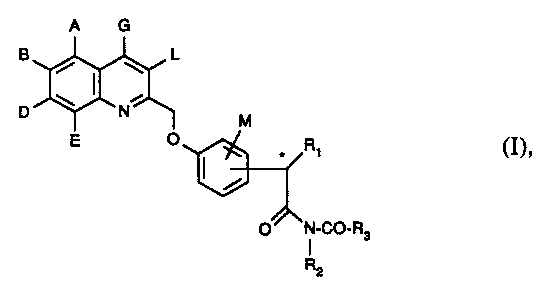
in welcher
- A, B, D, E, G, L und M: unabhängig voneinander
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
- R¹: für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
- R²: für Wasserstoff oder geradkettiges oder verzweigtes Alkyl mit bis zu 6 Kohlenstoffatomen oder Benzyl steht,
- R³: für geradkettiges oder verzweigtes Alkyl mit bis zu 8 Kohlenstoffatomen, Benzyl, Cycloalkyl mit 3 bis 12 Kohlenstoffatomen oder Phenyl steht, das gegebenenfalls durch Halogen, Nitro, Cyano oder Hydroxy substituiert ist, oder für eine Gruppe der Formel -NR⁴R⁵ steht,
worin
- R⁴ und R⁵: gleich oder verschieden sind und Wasserstoff, geradkettiges oder verzweigtes Alkyl mit bis zu 6 Kohlenstoffatomen, Benzyl oder Phenyl bedeuten,
gegebenenfalls in einer isomeren Form und deren Salze.

Überraschenderweise zeigen die erfindungsgemäßen Chinolylmethoxyphenylessigsäure-acylamide und -harnstoffe der allgemeinen Formel (I) eine hohe in vitro Aktivität als Leukotriensynthesehemmer.

Im Rahmen der vorliegenden Erfindung werden physiologisch unbedenkliche Salze bevorzugt. Physiologisch unbedenkliche Salze der Chinolylmethoxyphenylessigsäure-acylamide und -harnstoffe können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammmoniumsalze. Bevorzugt werden Natrium-, Kalium- und Ammoniumsalze.

Die erfindungsgemäßen Verbindungen existieren in stereoisomeren Formen (*), die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B, D, E, G, L und M: unabhängig voneinander
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,
- R¹: für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 6 Kohlenstoffatomen substituiert sind,
- R²: für Wasserstoff oder geradkettiges oder verzweigtes Alkyl mit bis zu 4 Kohlenstoffatomen steht,
- R³: für geradkettiges oder verzweigtes Alkyl mit bis zu 6 Kohlenstoffatomen, Benzyl, Cyclopropyl, Cyclopentyl, Cyclohexyl, Cycloheptyl oder Phenyl steht, das gegebenenfalls durch Fluor, Chlor, Brom oder Hydroxy substituiert ist,
oder für eine Gruppe der Formel -NR⁴R⁵ steht,
worin
- R⁴ und R⁵: gleich oder verschieden sind und Wasserstoff, geradkettiges oder verzweigtes Alkyl mit bis zu 4 Kohlenstoffatomen oder Benzyl bedeuten
gegebenenfalls in einer isomeren Form und deren Salze.

Besonders bevorzugt sind Verbindungen der allgemeinen Formel (I),
in welcher
- A, B, D, E, G, L und M: unabhängig voneinander
für Wasserstoff, Hydroxy, Fluor, Chlor, Brom oder geradkettiges oder verzweigtes Alkyl mit bis zu 4 Kohlenstoffatomen stehen,
- R¹: für Cyclopropyl, Cyclopentyl, Cyclohexyl oder Cycloheptyl steht, die gegebenenfalls durch geradkettiges oder verzweigtes Alkyl mit bis zu 4 Kohlenstoffatomen substituiert sind,
- R²: für Wasserstoff, Methyl, Ethyl oder Phenyl steht,
- R³: für geradkettiges oder verzweigtes Alkyl mit bis zu 4 Kohlenstoffatomen, Cyclopropyl, Cyclopentyl oder Cyclohexyl steht, oder
für eine Gruppe der Formel -NR⁴R⁵ steht,
worin
- R⁴ und R⁵: gleich oder verschieden sind und Wasserstoff, Methyl, Ethyl oder Benzyl bedeuten.

Ganz besonders bevorzugt sind Verbindungen der allgemeinen Formel (I),
in welcher
A, B, D, E, G, L und M für Wasserstoff stehen.

Ebenso sind solche ganz besonders bevorzugt, in denen der Rest 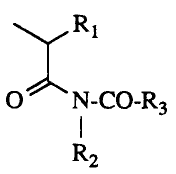
in 4-Stellung zum Chinolylmethoxyrest steht.

Außerdem wurden Verfahren zur Herstellung der erfindungsgemäßen Verbindungen der allgemeinen Formel (I) gefunden, dadurch gekennzeichnet, daß man
[A] Carbonsäuren der allgemeinen Formel (II) 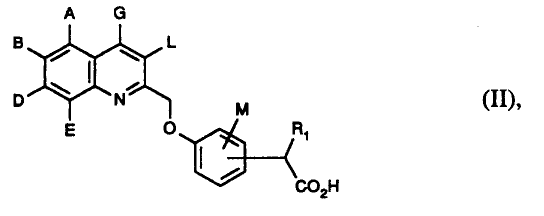 in welcher
   A, B, D, E, G, L, M und R¹ die oben angegebene Bedeutung haben,
   gegebenenfalls nach Aktivierung der Carbonsäurefunktion, beispielsweise über die Säurehalogenide oder -anhydride, mit Amiden der allgemeinen Formel (III)

   H₂N-CO-R³ (III),

   in welcher
   - R³: die oben angegebene Bedeutung hat
   in organischen Lösemitteln, gegebenenfalls in Anwesenheit von Basen umsetzt,
   oder
[B] im Fall, daß R³ für die Gruppe -NR⁴R⁵ steht, auch Amide der allgemeinen Formel (IV) 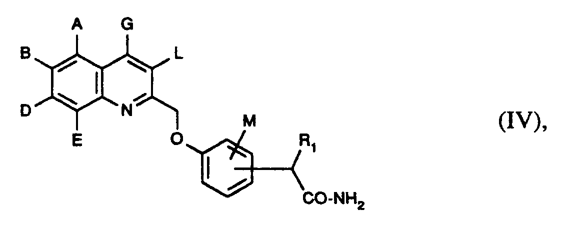 in welcher
   A, B, D, E, G, L, M und R¹ die oben angegebene Bedeutung haben,
   entweder mit Halogensulfonylisocyanaten der allgemeinen Formel (V) oder mit Harnstoffen der allgemeinen Formel (VI)

   T-SO₂-N=C=O (V) oder H₂N-CO-NR⁴R⁵ (VI),

   in welchen
   - R⁴ und R⁵: die oben angegebene Bedeutung haben und
   - T: für Halogen, vorzugsweise für Chlor steht,
   nach den unter [A] aufgeführten Reaktionsbedingungen kondensiert,
   oder
[C] die Verbindungen der allgemeinen Formel (IV) mit Acetalen der allgemeinen Formel (VII) 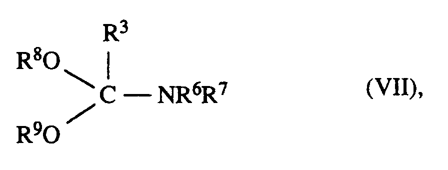 in welcher
   - R³: die oben angegebene Bedeutung hat und
   - R⁶, R⁷, R⁸ und R⁹: gleich oder verschieden sind und C₁-C₆Alkyl bedeuten,
   in Anwesenheit von Essigsäure umsetzt,
   und im Fall, daß R², R⁴ und/oder R⁵ nicht Wasserstoff bedeuten,
   gegebenenfalls auch eine Alkylierung nachträglich nach üblichen Methoden durchführt und die Substituenten A, B, D, E, G, L und M ebenfalls nach bekannten Methoden variiert,
   und im Fall der enantiomerenreinen Verbindungen die entsprechenden Säuren nach üblicher Methode trennt und anschließend wie oben aufgeführt weiter umsetzt.

Das erfindungsgemäße Verfahren kann durch folgendes Formelschema beispielhaft erläutert werden: 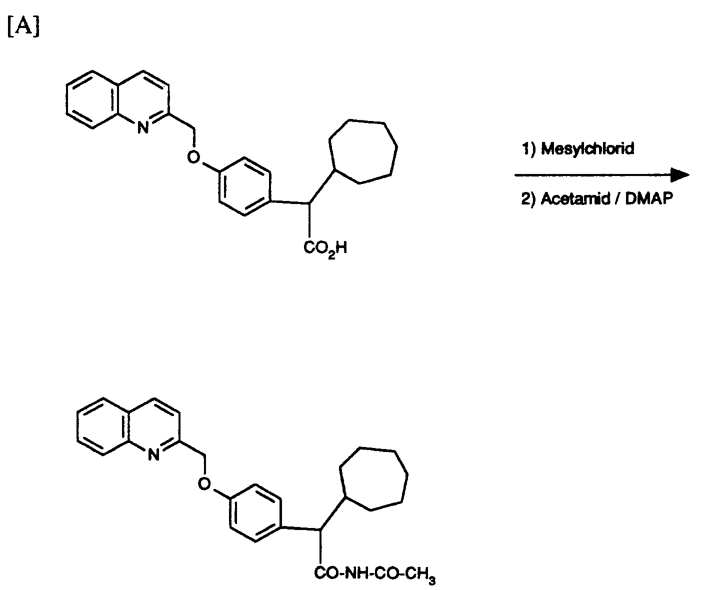 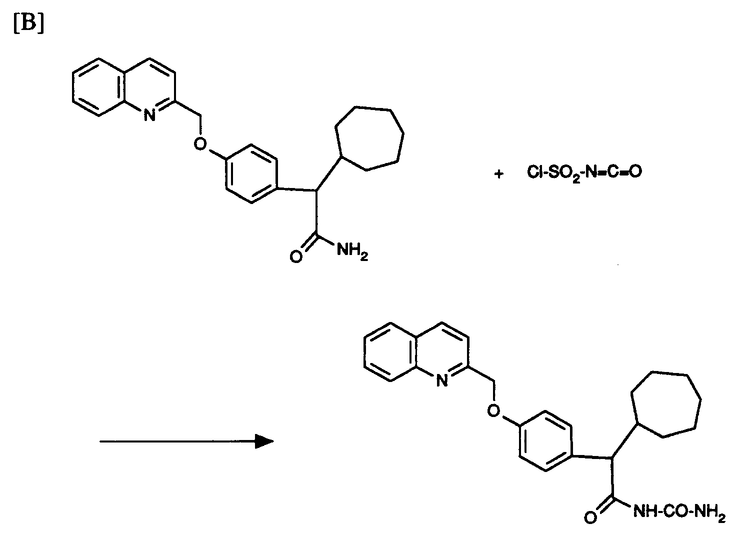
Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Dichlormethan. Bevorzugt sind Tetrahydrofuran, Aceton und Dimethylformamid.

Als Basen für die einzelnen Verfahrensschritte, insbesondere für die Amidierung und Acylierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Dimethylaminopyridin, Piperidin, Tetrabutylammoniumhydroxid, Pyridin, Triethylamin oder N-Methylpiperidin. Bevorzugt sind Kaliumcarbonat, Natriumhydrid, Piperidin, Triethylamin und Dimethylaminopyridon.

Die Amidierung und Acylierung werden im allgemeinen in einem Temperaturbereich von 0°C bis 150°C, bevorzugt bei 25°C bis 100°C, durchgeführt.

Die Amidierung und Acylierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Zur Aktivierung der Carbonsäure eignen sich die üblichen Reagenzien wie anorganische Halogenide, beispielsweise Thionylchlorid, Mesylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Im allgemeinen setzt man 1 bis 3 Mol, bevorzugt 1 bis 2 Mol, besonders bevorzugt 1 Mol Halogensulfonylisocyanat, bezogen auf 1 Mol des Amids ein.

Die Verbindungen der allgemeinen Formel (II) sind an sich bekannt oder können nach üblicher Methode hergestellt werden [vgl. DOS 38 18 443].

Die Verbindungen der allgemeinen Formel (III) sind bekannt oder können nach üblichen Methoden hergestellt werden [vgl. CA I 60-35-5].

Die Halogensulfonylisocyanate der allgemeinen Formel (V) sind ebenfalls bekannt.

Die Harnstoffe der allgemeinen Formel (VI) sind ebenfalls bekannt.

Die Verbindungen der allgemeinen Formel (IV) sind neu und können hergestellt werden, indem man die Säuren der allgemeinen Formel (II) ebenfalls nach Aktivierung der Carbonsäurefunktion wie oben beschrieben mit einem der oben aufgeführten Lösemittel, entweder im Ammoniakstrom oder durch Umsetzung mit den entsprechenden Aminen amidiert oder die entsprechenden 2-alkylierten 2-[4-(Chinolin-2-yl-methoxy)phenyl]-acetonitrile mit Säuren, beispielsweise Salzsäure, reduziert.

Das Verfahren wird im allgemeinen in einem Temperaturbereich von -10°C bis 120°C, bevorzugt von 25°C bis 100°C, und Normaldruck durchgeführt.

Die Verbindungen der allgemeinen Formel (VII) sind an sich bekannt (vgl. Beil. 4, 308).

Die erfindungsgemäßen Verbindungen können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Die Verbindungen der allgemeinen Formel (I) zeigen überraschenderweise eine hohe in vitro Aktivität als Leukotriensynthesehemmer und eine starke in vivo Wirkung nach oraler Applikation.

Sie sind somit bevorzugt zur Behandlung und Verhütung von Erkrankungen der Atemwege wie Allergien/Asthma, Bronchitis, Emphysema, Schocklunge, Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen Verbindungen können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die 5-Lipoxygenase-Hemmung in vitro wurde die Freisetzung von Leukotrien B₄ (LTB₄) an polymorphkernigen Humanleukozyten (PMN) nach Zugabe von Substanzen und Ca-Ionophor mittels reverse phase HPLC nach Borgeat, P. et al., Proc. Nat. Acad. Sci. 76, 2148-2152 (1979), bestimmt.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

### 2-[4-(Chinolin-2-yl-methoxy)phenyl]-acetonitril

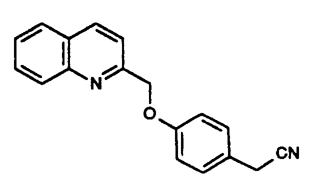

18 g (0,101 mol) Chinolin-2-methylchlorid, 13,3 g (0,1 mol) 4-Hydroxyphenylacetonitril und 14 g (0,1 mol) Kaliumcarbonat (pulverisiert und getrocknet bei 110°C) werden in 400 ml trockenem Aceton 72 Stunden zum Sieden erhitzt. Anschlieltend laßt man erkalten, filtriert vom Festprodukt ab und dampft das Filtrat i.V. zur Trockene ein. Man nimmt den Rückstand in 250 ml Dichlormethan auf, wäscht zweimal mit 250 ml 2 n Natronlauge, dann mit Wasser neutral, trocknet mit Natriumsulfat und dampft i.V. zur Trockene ein. Die Rekristallisation erfolgt aus Diisopropylether/Essigester.
Ausbeute: 21,6 g (78,8% der Theorie)
Fp.: 104 - 105°C (farblose Kristalle)

### Beispiel II

### 2-[4(Chinolin-2-yl-methoxy)phenyl]-2-cyclopentyl-acetonitril

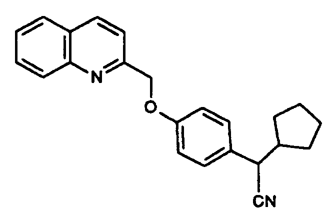

Man suspendiert bei 0°C 0,6 g (80%iges) Natriumhydrid (0,02 mol) in 40 ml abs. DMF und tropft 5,5 g (0,02 mol) der Verbindung aus Beispiel I in 20 ml DMF zu. Es setzt eine Wasserstoffentwicklung ein. Die Temperatur steigt dabei auf Raumtemperatur an. Man läßt noch 1 Stunde bei Raumtemperatur nachrühren, kühlt dann auf 0°C ab und tropft 3 g (0,02 mol) Cyclopentylbromid zu. Man läßt noch über Nacht reagieren, engt dann im Vakuum zur Trockene ein und rührt den Rückstand mit 180 ml Wasser / Dichlormethan (1:1) aus. Die organische Phase wird abgetrennt, getrocknet, auf ein kleines Volumen eingeengt und säulenchromatographisch getrennt (Kieselgel 60, Laufmittel: Toluol/Essigester = 9:1).
R_{f}=0,5
Ausbeute: 4 g (53% der Theorie)
Fp.: 87°C (farblose Kristalle)

### Beispiel III

### 2-[4-(Chinolin-2-yl-methoxy)phenyl]-2-cyclopentyl-essigsäureamid

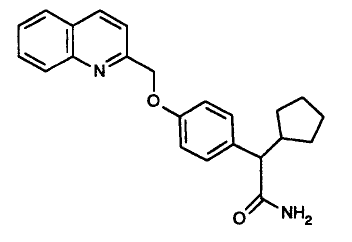

2 g (5,8 mmol) der Verbindung aus Beispiel II werden in 6 ml konz. Salzsäure suspendiert und über Nacht bei 40°C gerührt. Nach dem Abkühlen auf Raumtemperatur wird bis zur völligen Lösung THF zugesetzt und mit Natriumhydrogencarbonatlösung neutralisiert. Die organische Phase wird abgetrennt, mit Natriumsulfat getrocknet und im Vakuum auf ein kleines Volumen eingeengt. Die Trennung erfolgt säulenchromatographisch (Kieselgel 60, Laufmittel: Dichlormethan/Essigester/Eisessig = 80/15/15).
R_{f} = 0,35 (bei R_{f} ca. 0.68 liegt die Säure)
Ausbeute: 1,28 g (71,3% der Theorie)
Fp.: 178°C (farblose Kristalle)

### Herstellungsbeispiele

### Beispiel 1

### N-Acetyl-2[4-(Chinolin-2-yl-methoxy)phenyl]-2-cycloheptyl-essigsäureamid

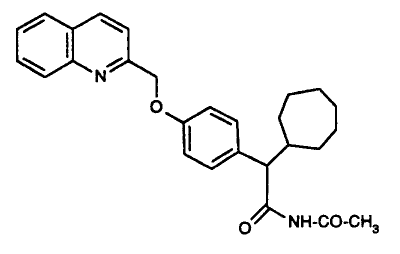

8,0 g (0,02 mol) 2-[4-(Chinolin-2-yl-methoxy)phenyl]-2-cycloheptyl-essigsäure und 4,0 g (5,6 ml/0,04 mol) Triethylamin werden in 80 mi THF gelöst, auf 0°C abgekühlt und mit 2,3 g (1,6 ml/0,02 mol) Mesylchlorid versetzt. Dazu gibt man bei 0°C 2,4 g (0,04 mol) Acetamid und 7,2 g (0,06 mol) Dimethylaminopyridin (DMAP), gelöst in 20 ml THF. Man läßt den Ansatz 48 h bei Raumtemperatur reagieren (rühren), engt dann i.V. zur Trockene ein und rührt mit 50 ml Wasser und 50 ml Dichlormethan den Rückstand aus. Die organische Phase wird abgetrennt, getrocknet, auf ein kleines Volumen eingeengt und säulenchromatographisch getrennt (Kieselgel 60, Laufmittel: Toluol/Essigester/Eisessig = 8/1/1).
Ausbeute: 0,8 g (9% der Theorie)
Fp.: 148°C (farblose Kristalle)

### Beispiel 2

### N-Carbamoyl-2-[4-(chinolin-2-yl-methoxy)phenyl]-2-cycloheptyl-essigsäureamid

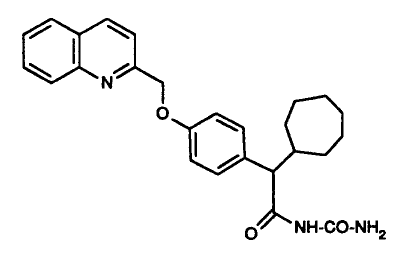

10 g (0,0257 mol) der Verbindung aus Beispiel III werden bei 0°C in 100 ml THF suspendiert und unter Feuchtigkeitsausschluß mit 2,5 ml (4 g/0,028 mol) Chlorsulfonylisocyanat versetzt. Man läßt 15 Minuten unter Rühren nachreagieren, versetzt mit 120 ml Eisessig/Wasser (2/1) und läßt die Temperatur auf Raumtemperatur ansteigen. Zur Vervollständigung der Reaktion erhitzt man noch 15 Minuten auf 100°C, wobei eine einheitliche Lösung entsteht. Die Lösung dampft man i.V. zur Trockene ein, rührt ihn 15 Minuten mit 50 ml Wasser aus und rekristallisiert den Rückstand aus THF/Ethylenchlorid.
Ausbeute: 8,0 g (72% der Theorie)
Fp.: 222°C (Zers.)(farblose Kristalle)

### Beispiele 3 und 4

### (+)-N-Carbamoyl-2-[4-(chinolin-2-yl-methoxy)phenyl]-2-cycloheptyl-essigsäureamid (3) (-)-N-Carbamoyl-2-[4-(chinolin-2-yl-methoxy)phenyl]-2-cycloheptyl-essigsäureamid (4)

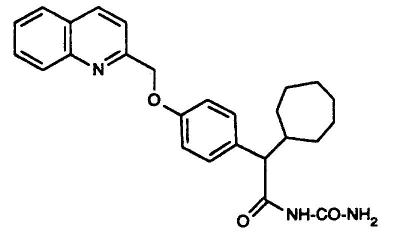

Analog der Vorschrift des Beispiels 2 wird das (+)- und das (-)-Enantiomer durch Umsetzen des enantiomerenreinen (+)- bzw. (-)-2-[4-(Chinolin-2-yl-methoxy)phenyl]-2-cycloheptyl-essigsäureamids mit Chlorsulfonylisocyanat erhalten.

## Patentansprüche

1. Chinolylmethoxyphenylessigsäure-acylamide und -harnstoffe der allgemeinen Formel 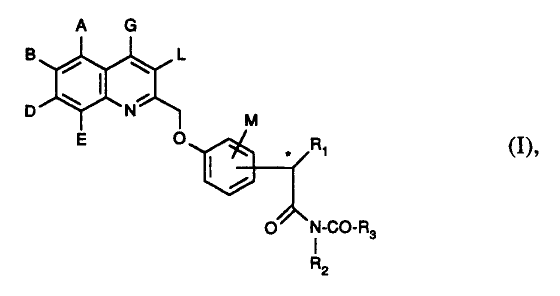 in welcher
A, B, D, E, G, L und M unabhängig voneinander
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
R¹ für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
R² für Wasserstoff oder geradkettiges oder verzweigtes Alkyl mit bis zu 6 Kohlenstoffatomen oder Benzyl steht,
R³ für geradkettiges oder verzweigtes Alkyl mit bis zu 8 Kohlenstoffatomen, Benzyl, Cycloalkyl mit 3 bis 12 Kohlenstoffatomen oder Phenyl steht, das gegebenenfalls durch Halogen, Nitro, Cyano oder Hydroxy substituiert ist,
oder für eine Gruppe der Formel -NR⁴R⁵ steht,
worin
R⁴ und R⁵ gleich oder verschieden sind und Wasserstoff, geradkettiges oder verzweigtes Alkyl mit bis zu 6 Kohlenstoffatomen, Benzyl oder Phenyl bedeuten,
gegebenenfalls in einer isomeren Form und deren Salze.

2. Chinolylmethoxyphenylessigsäure-acylamide und -harnstoffe nach Anspruch 1, wobei
A, B, D, E, G, L und M unabhängig voneinander
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,
R¹ für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 6 Kohlenstoffatomen substituiert sind,
R² für Wasserstoff oder geradkettiges oder verzweigtes Alkyl mit bis zu 4 Kohlenstoffatomen steht,
R³ für geradkettiges oder verzweigtes Alkyl mit bis zu 6 Kohlenstoffatomen, Benzyl, Cyclopropyl, Cyclopentyl, Cyclohexyl, Cycloheptyl oder Phenyl steht, das gegebenenfalls durch Fluor, Chlor, Brom oder Hydroxy substituiert ist,
oder für eine Gruppe der Formel -NR⁴R⁵ steht,
worin
R⁴ und R⁵ gleich oder verschieden sind und Wasserstoff, geradkettiges oder verzweigtes Alkyl mit bis zu 4 Kohlenstoffatomen oder Benzyl bedeuten
gegebenenfalls in einer isomeren Form und deren Salze.

3. Chinolylmethoxyphenylessigsäure-acylamide und -harnstoffe nach Anspruch 1, worin
A, B, D, E, G, L und M unabhängig voneinander
für Wasserstoff, Hydroxy, Fluor, Chlor, Brom oder geradkettiges oder verzweigtes Alkyl mit bis zu 4 Kohlenstoffatomen stehen,
R¹ für Cyclopropyl, Cyclopentyl, Cyclohexyl oder Cycloheptyl steht, die gegebenenfalls durch geradkettiges oder verzweigtes Alkyl mit bis zu 4 Kohlenstoffatomen substituiert sind,
R² für Wasserstoff, Methyl, Ethyl oder Phenyl steht,
R³ für geradkettiges oder verzweigtes Alkyl mit bis zu 4 Kohlenstoffatomen, Cyclopropyl, Cyclopentyl oder Cyclohexyl steht, oder
für eine Gruppe der Formel -NR⁴R⁵ steht,
worin
R⁴ und R⁵ gleich oder verschieden sind und Wasserstoff, Methyl, Ethyl oder Benzyl bedeuten.

4. Chinolylmethoxyphenylessigsäure-acylamide und -harnstoffe nach Anspruch 1, worin
A, B, D, E, G, L und M für Wasserstoff stehen.

5. Chinolylmethoxyphenylessigsäure-acylamide und -harnstoffe nach Anspruch 1 zur therapeutischen Anwendung.

6. Verfahren zur Herstellung von Chinolylmethoxyphenylessigsäure-acylamiden und -harnstoffen nach Anspruch 1, dadurch gekennzeichnet, daß man
[A] Carbonsäuren der allgemeinen Formel (II) 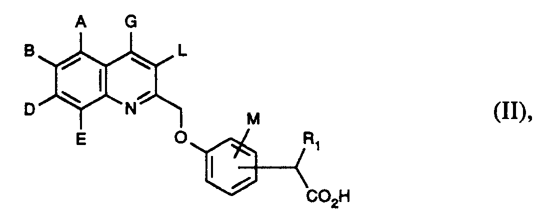 in welcher
A, B, D, E, G, L, M und R¹ die oben angegebene Bedeutung haben,
gegebenenfalls nach Aktivierung der Carbonsäurefunktion, beispielsweise über die Säurehalogenide oder -anhydride, mit Amiden der allgemeinen Formel (III)
H₂N-CO-R³ (III),
in welcher
R³ die oben angegebene Bedeutung hat
in organischen Lösemitteln, gegebenenfalls in Anwesenheit von Basen umsetzt,
oder
[B] im Fall, daß R³ für die Gruppe -NR⁴R⁵ steht, auch Amide der allgemeinen Formel (IV) 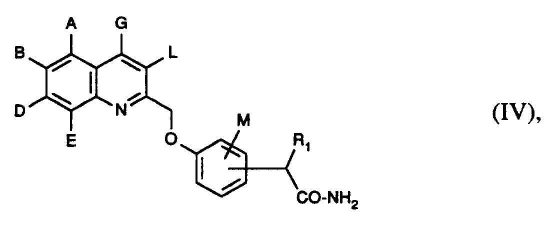 in welcher
A, B, D, E, G, L, M und R¹ die oben angegebene Bedeutung haben,
entweder mit Halogensulfonylisocyanaten der allgemeinen Formel (V) oder mit Harnstoffen der allgemeinen Formel (VI)
T-SO₂-N=C=O (V) oder H₂N-CO-NR⁴R⁵ (VI),
in welchen
R⁴ und R⁵ die oben angegebene Bedeutung haben und
T für Halogen, vorzugsweise für Chlor steht,
nach den unter [A] aufgeführten Reaktionsbedingungen kondensiert,
oder
[C] die Verbindungen der allgemeinen Formel (IV) mit Acetalen der allgemeinen Formel (VII) 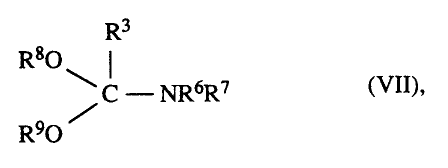 in welcher
R³ die oben angegebene Bedeutung hat und
R⁶, R⁷, R⁸ und R⁹ gleich oder verschieden sind und C₁-C₆Alkyl bedeuten,
in Anwesenheit von Essigsäure umsetzt,
und im Fall, daß R², R⁴ und/oder R⁵ nicht Wasserstoff bedeuten,
gegebenenfalls auch eine Alkylierung nachträglich nach üblichen Methoden durchführt und die Substituenten A, B, D, E, G, L und M ebenfalls nach bekannten Methoden variiert,
und im Fall der enantiomerenreinen Verbindungen die entsprechenden Säuren nach üblicher Methode trennt und anschließend wie oben aufgeführt weiter umsetzt.

7. Arzneimittel enthaltend mindestens ein Chinolylmethoxyphenylessigsäureacylamid oder -harnstoff nach Anspruch 1.

8. Verfahren zur Herstellung eines Arzneimittels nach Anspruch 7, dadurch gekennzeichnet, daß man die Chinolylmethoxyphenylessigsäure-acylamide und -harnstoffe gegebenenfalls mit Hilfe von üblichen Hilfs- und Trägerstoffen in eine geeignete Applikationsform überführt.

9. Verwendung von Chinolylmethoxyphenylessigsäure-acylamiden und -harnstoffen nach Anspruch 1 zur Herstellung von Arzneimitteln.

10. Verwendung von Chinolylmethoxyphenylessigsäure-acylamiden und -harnstoffen nach Anspruch 1 zur Herstellung von Leukotriensynthesehemmern.
